# EUROPÄISCHE PATENTANMELDUNG

(11) **EP 1 190 629 A1**
(43) Veröffentlichungstag der Anmeldung: **27.03.2002**
(21) Anmeldenummer: 00810846.6
(22) Anmeldetag: 18.09.2000
(51) Int. Cl.: A23L 1/30, A23L 1/302, A61K 31/20

(54) **Omega-3 Fettsäuren mit Vitamin E-Nahrungskonzentrat**

(71) Anmelder: EnergyBalance AG, 9491 Ruggell (LI)
(72) Erfinder:

(57) **Zusammenfassung**

Medizinische Untersuchungen haben gezeigt, dass die Kombination von mehrfach ungesättigter essentieller Fettsäure Omega-3 zusammen mit Vitamin E besondere therapeutische Effekte hat.

Bisher wurden als Vitamin E-Rohstoffe chemisch isolierte Substanzen in Form von Tocopherol-Verbindungen verwendet. Vitamin E Vergleichsstudien haben jedoch gezeigt, dass die Resorption und Bioverfügbarkeit von Vitamin E-Leguminoseextrakt, einem Nahrungskonzentrat mit standardisiertem Vitamingehalt, höher ist als diejenige von Tocopherol-Verbindungen.

Die Kombination von Omega-3 mit Vitamin E-Nahrungskonzentrat verbessert die therapeutische Wirkung.

## Beschreibung

Medizinische Untersuchungen haben gezeigt, dass die Kombination von mehrfach ungesättigter essentieller Fettsäure Omega-3 zusammen mit Vitamin E besondere therapeutische Effekte hat.

Bisher wurden als Vitamin E-Rohstoffe chemisch isolierte Substanzen in Form von Tocopherol-Verbindungen verwendet. Vitamin E Vergleichsstudien haben jedoch gezeigt, dass die Resorption und Bioverfügbarkeit von Vitamin E-Leguminoseextrakt, einem Nahrungskonzentrat mit standardisiertem Vitamingehalt, höher ist als diejenige von Tocopherol-Verbindungen.

Die Kombination von Omega-3 mit Vitamin E-Nahrungskonzentrat verbessert die therapeutische Wirkung.

## Patentansprüche

1. Mehrfach ungesättigte Fettsäure Omega-3, oder deren Bestandteile Eicosapentaensäure und/oder Docosahexaensäure, welche in Kombination mit Vitaminen, in jeder Form medizinische, therapeutische, präventive und nahrungsergänzende Anwendung bei Mensch und Tier findet,
**dadurch gekennzeichnet,**
**dass** die Vitamine Nahrungskonzentrate sind.

2. Mehrfach ungesättigte Fettsäure Omega-3, oder deren Bestandteile Eicosapentaensäure und/oder Docosahexaensäure, welche in Kombination mit Vitaminen, in jeder Form medizinische, therapeutische, präventive und nahrungsergänzende Anwendung bei Mensch und Tier findet,
**dadurch gekennzeichnet,**
**dass** die Vitamine Nahrungskonzentrate mit standardisiertem Vitamingehalt sind.

3. Mehrfach ungesättigte Fettsäure Omega-3, oder deren Bestandteile Eicosapentaensäure und/oder Docosahexaensäure, welche in Kombination mit Vitamin E, in jeder Form medizinische, therapeutische, präventive und nahrungsergänzende Anwendung bei Mensch und Tier findet,
**dadurch gekennzeichnet,**
**dass** das Vitamin E ein Leguminoseextrakt ist.

4. Mehrfach ungesättigte Fettsäure Omega-3, oder deren Bestandteile Eicosapentaensäure und/oder Docosahexaensäure, welche in Kombination mit Vitamin E und D, in jeder Form medizinische, therapeutische, präventive und nahrungsergänzende Anwendung bei Mensch und Tier findet,
**dadurch gekennzeichnet,**
**dass** das Vitamin E ein Leguminoseextrakt und das Vitamin D ein Hefepulver ist.
